# EUROPEAN PATENT APPLICATION

(11) **EP 2 166 042 A1**
(43) Date of publication of application: **24.03.2010**
(21) Application number: 08765360.6
(22) Date of filing: 09.06.2008
(51) Int. Cl.: C09C 3/06, A61K 8/19, A61K 8/25, A61K 8/26, A61Q 1/00, A61Q 1/10, C08K 9/02, C08L 101/00, C09C 1/30, C09C 1/40, C09C 1/42, C09D 7/12, C09D 11/00

(54) **BLACK GLITTER, AND COSMETICS, COATING MATERIALS, INK OR RESIN COMPOSITIONS CONTAINING THE GLITTER**

(30) Priority: 08.06.2007 JP 2007153170
(71) Applicant: Nippon Sheet Glass Company Limited, Tokyo 108-6321 (JP)
(72) Inventor: KITMAMURA, Takeaki, Tokyo, 108-6321 (JP); NIKI, Hiroyuki, Tokyo, 108-6321 (JP)
(74) Representative: Hall, Matthew Benjamin
(86) International application number: PCT/JP2008/060570
(87) International publication number: WO 2008/150011

(57) **Abstract**

The black bright pigment of the present invention includes a scaly inorganic base 10 and a coating 20 covering the inorganic base 10 and containing nanoparticles including a pure substance of metal element other than copper or a noble-metal alloy (free of copper) and the nanoparticles include crystal dendrites. The average particle size of the nanoparticles is preferably 20 to 50 nm. The average width of the crystal dendrites is preferably 10 nm or less. The pure substance of noble metal or noble metal contained in the noble-metal alloy is preferably at least one selected from the group consisting of silver, gold, platinum, palladium and iridium.

## Description

### Technical Field

The present invention relates to a black bright pigment, and further to a cosmetic, paint, ink or a resin composition containing the black bright pigment.

### Background Art

Conventionally, as metallic pigments, a flaky aluminum powder, natural mica chip particles or graphite chip particles covered with a metallic oxide such as titanium dioxide or iron oxide or iron oxide particles having α-iron oxide crystal particles as the main ingredient have been known. These metallic pigments have the property of glittering by reflecting light from the surfaces thereof, and are used as materials for paint, ink, resin compositions for molding, and cosmetics. These bright pigments provide a coating surface obtained by applying paint, a printing surface using ink, or the surface of a resin molding molded using a resin composition for molding with a unique outer appearance having a wide variety and being excellent in beauty, in combination with the color tone of a base material of the surface. Thus, the metallic pigments have been used widely in accordance with such application purposes as for automobiles, motorcycles, OA equipment, mobile phones, household electrical appliances, a variety of printed matters and writing instruments.

Among the conventional metallic pigments, there are only a small number of metallic pigments that present a jet-blackish appearance. As an example of such metallic pigments, a jet-blackish metallic pigment has been disclosed (see Patent document 1, for example). In the jet-blackish metallic pigment, a glass powder is covered with a coating composed of a nickel alloy and having a thickness of 0.04 µm or less. The nickel alloy coating is, for example, a nickel-phosphorus alloy coating, where the weight ratio (P/Ni) of phosphorus (P) to nickel (Ni) is 0.1 to 0.2.

Further, a black pigment having inorganic anti-bacterial and anti-fungal functions has been proposed (see Patent document 2, for example). In the black pigment, a sliver alloy or a nickel alloy is deposited on the surfaces of particles that have a particle size of 50 µm or less such that the primary particle size becomes 0.01 to 0.5 µm. The coating composed of the silver alloy or the nickel alloy is formed by physical vapor deposition, for example. The coating composed of the silver alloy contains, for example, 10 to 50 mass% of Cu and/or Zn and the coating composed of the nickel alloy contains, for example, 10 to 50 mass% of Cu and/or Sn.
Patent document 1: JP 2002-30232 A
Patent document 2: JP H11-181327A

### Disclosure of Invention

### Problem to be Solved by the Invention

However, the blackness of the pigments described in Patent documents 1 and 2 is not high and thus is insufficient. This is because the metals contained in the coatings are susceptible to oxidizing, so that the color phase takes on a red tinge.

With the foregoing in mind, the present invention provides a bright pigment with high blackness and a cosmetic, paint, ink or a resin composition containing the bright pigment.

### Means for Solving Problem

The black bright pigment of the present-invention includes a scaly inorganic base; and a coating covering the inorganic base and containing nanoparticles including a pure substance of noble metal other than copper or a noble-metal alloy (free of copper). The nanoparticles include crystal dendrites.

The cosmetic of the present invention includes the black bright pigment of the present invention.

The paint of the present invention includes the black bright pigment of the present invention.

The ink of the present invention includes the black bright pigment of the present invention.

The resin composition of the present invention includes the black bright pigment of the present invention.

A method of producing the black bright pigment of the present invention includes the steps of: coating a scaly inorganic base with a metal-containing film including a pure substance of noble metal other than copper or a noble-metal alloy (free of copper); and heating the inorganic base coated with the metal-containing film in an atmosphere of 350 to 550°C to make the metal-containing film into a coating containing nanoparticles having crystal dendrites on surfaces thereof.

### Brief Description of Drawings

[FIG. 1] FIG. 1 is a schematic cross-sectional view showing an example of the black bright pigment of the present invention.
[FIG. 2] FIG. 2 is an image of a black bright pigment of Example 1, taken with a Scanning electron microscope.
[FIG. 3] FIG. 3 is an image of a bright pigment of Comparative Example 2, taken with a scanning electron microscope.
[FIG. 4] FIG. 4 is an image of a bright pigment of Comparative Example 3, taken with a scanning electron microscope.

### Description of the Invention

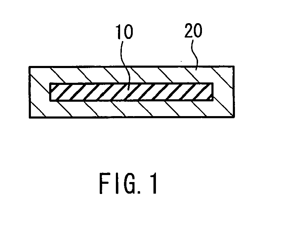

FIG. 1 is a schematic cross-sectional view showing an example of the black bright pigment of the present invention. As shown in FIG.1, the black bright pigment includes a scaly inorganic base 10 and a black-translucent coating 20 that covers the inorganic base 10. The coating 20 is composed of nanoparticles (generally referred to particles with a particle size of 100 nm or less). On the surfaces of the nanoparticles, crystal dendrites are formed

### Inorganic Base

For example, the inorganic base is substantially composed of at least one material selected from the group consisting of glass, natural mica, synthetic mica, silica and alumina. Among these materials, glass is preferable because it has high transparency and a smooth surface is likely to be obtained.

Although the method of producing the scaly glass base as an example of the inorganic base 10 is not particularly limited, for example, a blowing method is preferable. In the blowing method, first, a raw cullet is melted. The molten glass is discharged continuously from a circular slit, and at the same time, a gas such as air is blown through a blow nozzle attached to the interior of the circular slit. As a result, the molten glass is stretched and expanded to form a balloon. The balloon shaped glass is crushed to obtain the scaly glass base.

Examples of commercially available products of such a scaly glass base include MICROGLAS (registered trademark) and GLASFLAKE (registered trademark) series (RCF-160, REF-160, RCF-015, REF-015) manufactured by Nippon Sheet Glass Co., Ltd.

Although the shape of the inorganic base varies depending on how the inorganic base is applied, generally it is preferable that the inorganic base has an average particle size of 1 to 500 µm and an average thickness of 0.1 to 10 µm. In a case where the inorganic base is composed of glass and its particle size is too large, the black bright pigment gets crushed when it is mixed into paint or a resin composition. As a result, alkali components contained the glass may be dispersed in the paint or the resin composition. In contrast, in a case where the particle size of the inorganic base is too small, the principle surfaces of pieces of the bright pigment in the paint or the resin composition face random directions, so that light reflected by individual particles weakens and the brightness may be compromised. When the inorganic base has an average particle size of 1 to 500 µm, it is possible to prevent the black bright pigment from getting crushed during the mixing process.

In the present specification, the average particle size (D50) of the inorganic base corresponds to 50% of the cumulative volume in the particle size distribution. The particle size distribution is an index showing the sizes (particle size) and percentages of particles contained in a sample particle group serving as a measuring object and is measured on the basis of the laser diffraction scattering method. In the laser diffraction scattering method, the particle size distribution is determined by utilizing scattered light at the time of irradiating light to the particles.

### Coating

Although the nanoparticles constituting the coating 20 are substantially composed of a pure substance of noble metal other than copper or a noble-metal alloy (free of copper), they may contain an extremely small amount of impurities. As the pure substance of noble metal or noble metal contained in the noble-metal alloy, at least one noble metal selected from the group consisting of silver, gold, platinum, palladium and iridium is preferable. This is because these elements are resistant to oxidizing and a black bright pigment with high blackness that does not take on a tinge such as red can be obtained.

Examples of the noble-metal alloy include binary alloys such as a silver-gold alloy, a silver-platinum alloy, a silver-palladium alloy, a silver-iridium alloy, a gold-platinum alloy, a gold-palladium alloy, a gold-iridium alloy, a platinum-palladium alloy, a platinum-iridium alloy and a palladium-indium alloy, ternary alloys such as a slivergold-palladium alloy, a sliver-platinumpalladium alloy, a silver-gold-platinum alloy, a silver-gold-iridium alloy, a silverplatinum-iridium alloy, a gold-platinum-palladium alloy, a gold-platinum-iridium alloy and a platinum-palladium-indium alloy, quaternary alloys such as a silvergold-platinum-palladium alloy, a sliver-gold-platinum-iridium alloy, a silverplatinum-palladium-iridium alloy, a silver-gold-palladium-iridium alloy and a gold-platinum-palladium-iridium alloy, and quinary alloys such as a silver-gold-platinum-palladium-iridium alloy.

As described above, since the nanoparticles do not contain metal with a minus ΔG (Gibbs free energy change) value and a large absolute value, in other words, they do not contain Ni (ΔG = -420 kJ/mol) or Cu (ΔG = -250 kJ/mol) that is susceptible to oxidizing and they are composed of a pure substance of noble metal such as silver (ΔG = -10 kJ/mol), gold (ΔG = 20 kJ/mot), platinum (ΔG = 10 kJ/mol), palladium (AG = -100 kJ/mol) or iridium (AG = -10 kJ/mol) or a noble-metal alloy containing the listed pure substances of noble metal. Therefore, the coating 20 is prevented from taking on a red tinge caused by oxidization.

The nanoparticles contained in the coating are substantially crystal particles of a pure substance of noble metal other than copper or a noble-metal alloy (free of copper) grown during the formation process of the coating 20. In terms of increasing visible light absorption and reducing the reflectivity so as to achieve a jet-black appearance, in other words, to obtain a bright pigment with high blackness, the average particle size of the nanoparticles is preferably 20 to 50 nm and more preferably 20 to 40 nm. The average particle size of the nanoparticles can be measured by a method described in Examples below.

The crystal dendrites are grown on the surface of the coating 20 during the formation process of the coating 20 and substantially are composed of a pure substance of noble metal other than copper or a noble-metal alloy (free of copper). It is preferable that the average width of the crystal dendrites is 10 nm or less. This is because a black bright pigment with higher blackness can be obtained with such an average width. The average width of the crystal dendrites can be measured by a method described in Examples below.

Since the nanoparticles have the crystal dendrites on their surfaces, they have asperities like the surface of a small candy made by crystallizing sugar. On the surface of the coating containing such nanoparticles, scattering and repeated reflection (multiple reflection) occur. It is believed that an improvement in the blackness results from those phenomenon. Due to the synergy between the improvement in the blackness and the prevention of taking on a red tinge, the black bright pigment of the present embodiment presents high blackness.

Accordingly, by using the black bright pigment of the present embodiment, it is possible to provide a cosmetic, ink, paint, an artificial marble molded product, coated paper, etc. that present high blackness while being prevented from taking on a tinge such as red.

The black bright pigment of the present embodiment can be formed by the following method, for example.

First, a scaly inorganic base is covered with a metal-containing film including a pure substance of noble metal other than copper or a noble-metal alloy (free of copper). The metal-containing film includes nanoparticles. Next, by heating the inorganic base covered with the metal-containing film in an atmosphere at a predetermined temperature, crystal dendrites are formed on the surfaces of the nanoparticles. It should be noted that the average particle size of the nanoparticles contained in the metal-containing film does not virtually change even after the heating.

Although the method of forming the film is not particularly limited, a method such as spattering, chemical-vapor deposition or electroless (chemical) plating may be used. Among these methods, electroless plating is preferable because a uniform film can be formed easily on the scaly inorganic base.

For example, one of the following compounds can be used as the metal material of a plating solution used in electroless plating.
(1) silver material: silver nitrite
(2) gold material sodium gold(I) sulfite, gold(III) chloride[tetrachloro-gold(III) tetrahydrate]
(3) platinum material: platinum(IV) chloride[hexachloro platinum(IV) hexahydrate], potassium platinum(IV) chloride, sodium platinum(IV) chloride, sodium hexahydroxyplatinum(IV) hydrate, potassium platinum(II) chloride, dinitro diamine platinum(II), potassium tetranitroplatinum(II), tetraaminedichloro platinum(II)
(4) palladium material: dinitrodiamine palladium(II) (diamino-palladilum nitrite), palladium(II) chloride, sodium palladium(II) chloride, palladium(II) nitrite dihydrate, dichlorotetraamine palladium(II) monohydrate (tetraamine palladium(II) dichloride), dichlorodiamine palladium(II)
(5) iridium material: iridium(IV) chloride[hexachloroiridium(IV) hexahydrate], potassium iridium(IV) chloride, potassium iridium(III) chloride, mdium(IU) chloride

The atmosphere in which the inorganic base covered with the metal-containing film is heated needs be 350 to 550°C. When the temperature is lower than 350°C, the crystal dendrites will not be formed. Further, when the temperature is higher than 550°C, the crystal dendrites will also not be formed. The temperature of the atmosphere is preferably 350 to 550°C because the blackness improves. Although the heating time is not particularly limited so long as the crystal dendrites are formed on the surfaces of the nanoparticles, the heating time is preferably 1 to 10 hours and more preferably 2 to 6 hours because the blackness improves.

### Paint

Next, an example of the paint containing the black bright pigment of the present invention will be described. The application areas of the paint of the present embodiment include the exterior of automobiles, motorcycles and bicycles, buildings, tiles, furniture, utensils, containers, office supplies, sporting goods and the like. The paint of the present embodiment contains, for example, the black bright pigment of the present invention, a resin and a solvent.

At some intermediate point of a circulating line for cyclically using paint, a filter for removing foreign substances is provided. Paint is used cyclically in the painting process of car panels, for example. When a pigment with a large particle size is contained in paint, the pigment is captured by the filter, resulting in an increase in pressure loss during the application or a reduction in the content of a bright pigment in the paint. This adversely affects the coating quality. The black bright pigment contained in the paint preferably has an average particle size of 1 to 50 µm and an average thickness of 0.1 to 3 µm. This is because the adverse impacts on the coating quality caused by the filtration become small and pieces of the black bright pigment align neatly within a coat without sticking out from the coat surface, making the finished quality of the coating favorable.

Examples of the resin include thermosetting resins, such as an acrylic resin, a polyester resin, an epoxy resin, a phenol resin, a urea resin, a fluorocarbon resin, a polyester-urethane curable resin, an epoxy-polyester curable resin, an acrylic-polyester based resin, an acrylic-urethane curable resin, an acrylic-melamine curable resin and a polyester-melamine curable resin, and thermoplastic resins, such as a polyethylene resin, a polypropylene resin, a petroleum resin, a thermoplastic polyester resin and a thermoplastic fluorocarbon resin.

Examples of the solvent include: aliphatic hydrocarbons (for example, hexane, heptane, octane, etc); esters (for example, ethyl acetate, n-butyl acetate, isobutyl acetate, n-butyl acetate, etc.); ketones (for example, acetone, methyl ethyl ketone, methyl isobutyl ketone, etc); ethers (for example, diethyl ether, dioxane, tetrahydrofuran, etc); cellosolves (for example, methyl cellosolve (ethylene glycol monomethyl ether), ethyl cellosolve, propyl cellosolve, butyl cellosolve, phenyl cellosolve, benzyl cellosolve, etc); and carbitols (for example, diethylene glycol monomethyl ether, carbitol (diethylene glycol monoethyl ether), diethylene glycol monopropyl ether, etc.). They may be used in a combination of two or more kinds.

Although the content of each component in the paint is not limited in the present invention and it can be similar to that in conventionally known paint, the content of the black bright pigment is preferably 0.1 to 30 mass% and more preferably 1 to 20 mass% in a dry-cured coat, for example.

In terms of improving the efficiency of coating adhesion, the content of the resin in the paint is preferably 20 to 80 mass% and more preferably 30 to 60 mass% in a dry-cured coat.

The content of the solvent in the paint is not particularly limited and it may be set in accordance with the application method of the paint. However, when the application method is spray coating, for example, it is preferable to determine the content of the solvent such that the viscosity of the paint at 20°C becomes 10 to 30 Pa·s.

The paint of the present embodiment may contain a curing agent as needed Examples of the curing agent include polyisocyanate, amine, polyamide, polybasic acid, acid anhydride, polysulfide, trifluoroboric acid, acid dihydrazide and imidazole.

The paint of the present embodiment further may contain a pigment other than the black bright pigment of the present invention, a surfactant, a lubricant, an antifoaming agent, a leveling agent, etc.

### Resin composition

Next, an example of the resin composition containing the black bright pigment of the present invention will be described. Examples of molded products that can be molded using the resin composition of the present embodiment include cosmetic containers, food containers, wall materials, floor material, cases for household electric appliances, accessories, stationeries, toys, bathtubs, bath equipment, shoes, sporting goods, toilet goods and the like. The resin composition of the present embodiment contains the black bright pigment of the present invention and a base resin.

In terms of enhancing glittering on the surfaces of molded products molded using the resin composition, the inorganic base constituting the black bright pigment contained in the resin composition preferably has an average particle size of 0.01 to 2 mm and an average thickness of 0.5 to 30 µm.

Examples of the base resin include thermosetting resins, such as an acrylic resin, a polyester resin, an epoxy resin, a phenol resin, a urea resin, a fluorocarbon resin, a polyester-urethane curable resin, an epoxy-polyester curable resin, an acrylic-polyester based resin, an acrylic-urethane curable resin, an acxylic-melamine curable resin and a polyester-melamine curable resin, and thermoplastic resins, such as a polyethylene resin, a polypropylene resin, a petroleum resin, a thermoplastic polyester resin and a thermoplastic fluorocarbon resin. When a thermoplastic resin is used as the base resin, injection molding can be carried out. Thus, molded products having a complex shape can be molded. As the inorganic base, glass that does not have cleavage as possessed by mica is preferable because it can maintain the same particle size even after the injection molding.

The resin composition of the present embodiment may contain a curing agent as needed. Examples of the curing agent include polyisocyanate, amine, polyamide, polybasic acid, acid anhydride, polysulfide, trifluoroboric acid, acid dihydrazide, imidazole, etc.

The resin composition of the present embodiment further may contain a pigment other than the black bright pigment of the present invention, a surfactant, a lubricant, an antifoaming agent, a leveling agent, etc.

The content of each component in the resin composition is not limited in the present invention, and it can be similar to that in conventionally known resin compositions. However, in terms of enhancing the jet-blackish color (blackness) of molded products molded using the resin composition, the content of the black bright pigment is preferably 0.01 to 10 mass% and more preferably 0.5 to 5 mass%.

### Ink

Next, an example of the ink containing the black bright pigment of the present invention will be described. The ink of the present embodiment can be used as ink for writing instruments such as a variety of ballpoint pens and marking pens and printing ink such as gravure ink and offset ink. The ink of the present embodiment contains the black bright pigment of the present invention and a vesicle.

In terms of allowing a smooth handwriting surface, the black bright pigment contained in the ink for writing instruments preferably has an average particle size of 10 to 120 µm and an average thickness of 0.1 to 2 µm.

It is preferable that the black bright pigment contained in the printing ink has an average particle size of 10 to 50 µm and an average thickness of 0.1 to 2 µm. This is because plate fogging, contamination of an impression cylinder and plate clogging become less likely to occur, so that the printing suitability becomes favorable. It should be noted that the plate fogging is a phenomenon in which a doctor blade cannot sufficiently scrape off ink on the plate at the time of printing and the ink is transferred to impression paper, causing scumming of printed matters.

Examples of the vehicle for the ink for writing instruments include a mixture obtained by mixing at least one selected from the group consisting of an acrylic resin, a styrene-acrylic copolymer, polyvinyl alcohol, polyacrylate, an acryl-vinyl acetate copolymer, a microbe polysaccharide such as xanthan and a water-soluble vegetable polysaccharide such as guar with a solvent. Examples of the solvent include water, alcohol, hydrocarbon, ester, etc.

Examples of the vehicle for the gravure ink include at least one selected from the group consisting of gum rosin, wood rosin, talloil rosin, lime rosin, rosin ester, a maleate resin, a polyamide resin, a vinyl resin, nitrocellulose, cellulose acetate, ethyl cellulose, chlorinated rubber, cyclized rubber, an ethylene-vinyl acetate copolymer, an urethane resin, a polyester resin, an alkyd resin, gilsonite, dammar and shellac, a watersoluble resin obtained by imparting water solubility to one selected from the resins in the above group and a mixture obtained by mixing an oil-in-water (O/W) type resin with a solvent. Examples of the solvent include hydrocarbon, alcohol, ether, ester, water, etc.

Examples of the vehicle for the offset ink include a mixture obtained by mixing a resin such as a rosin-modified phenolic resin, a petroleum resin or an alkyd resin with a solvent. Example of the solvent include plant oils such as linseed oil, tung oil and soybean oil, n-paraffin, isoparaffin, aromatic, naphthene, α-olefin, water, etc.

Each of the above-described vehicles further may contain a dye, a pigment other than the black bright pigment of the present invention, a surfactant, a lubricant, an antifoaming agent, a leveling agent, etc.

The content of each component in the ink may vary in accordance with the application purpose of the ink. The content is not limited in the present invention and it may be similar to that in conventionally known ink.

### Cosmetic

Next, the cosmetic including the black bright pigment of the present invention will be described. The type of the cosmetic of the present embodiment is not particularly limited. Examples of the cosmetic include: nail cosmetics such as nail color and nail coat; eye and eyebrow cosmetics such as eye shadow, eyeliner, mascara and an eyebrow pencil; eyeliner pencils; makeup cosmetics of a sedimentation type that are kept in a state where a lame agent is sedemented in water or a solvent other than water but are adequately shaken at the time of use; and the like.

The cosmetic of the present embodiment is obtained by mixing the bright pigment of the present invention with a medium, if needed, with the use of a known means.

In a case where the cosmetic of the present embodiment is a nail cosmetic such as nail color, in terms of achieving both high brightness and favorable applicability, the content of the black bright pigment of the present invention is preferably 0.1 to 50 mass% and more preferably 3 to 40 mass%, when the total mass of the cosmetic is 100 mass%.

In a case where the cosmetic of the present embodiment is a solid powder cosmetic such as eye shadow obtained by drying a powder after dry filling the powder using a press or the like or wet filling the powder using a volatile solvent, in terms of achieving both high brightness and favorable feeling in use, the content of the black bright pigment of the present invention is preferably 5 to 80 mass% and more preferably 10 to 60 mass%, when the total mass of the cosmetic is 100 mass%.

In a case where the cosmetic of the present embodiment is a powder cosmetic such as a loose powder, the cosmetic is mixed with sebum present on the skin at the time of use. Thus, the content of the black bright pigment of the present invention is preferably 70 to 100 mass%, when the total mass of the cosmetic is 100 mass%.

In a case where the cosmetic of the present embodiment is an oily solid cosmetic such as oily eye shadow, in terms of sufficiently ensuring the effect of the black bright pigment of the present invention as a lame agent as well as ensuring the favorable formability of the cosmetic, the content of the black bright pigment of the present invention is preferably 1 to 60 mass% and more preferably 3 to 50 mass%, when the total mass of the cosmetic is 100 mass%.

In a case where the cosmetic of the present embodiment is an emulsion makeup cosmetic obtained by emulsifying a water phase and an oil phase with an activator, in terms of sufficiently ensuring the effect of the black bright pigment of the present invention as a lame agent as well as ensuring high stability of the emulsion, the content of the black bright pigment of the present invention is preferably 1 to 50 mass% and more preferably 3 to 40 mass%, when the total mass of the cosmetic is 100 mass%.

In a case where the cosmetic of the present embodiment is an aqueous makeup cosmetic, in terms of sufficiently ensuring the effect of the black bright pigment of the present invention as a lame agent as well as ensuring favorable feeling in use, the content of the black bright pigment of the present invention is preferably 0.1 to 60 mass% and more preferably 1 to 40 mass%, when the total mass of the cosmetic is 100 mass%. Examples of the aqueous makeup cosmetic include aqueous mascara containing water, a watersoluble resin or an oil-in-water (O/W) type resin and a thickener.

Although the medium contained in the cosmetic of the present invention varies depending on the type of cosmetic, examples of the medium include: ketones that are in a liquid state at room temperature, such as methyl ethyl ketone, methyl isobutyl ketone, diisobutyl ketone, isophorone, cyclohexanoate and acetone; alcohols that are in a liquid state at room temperature, such as ethanol, isopropanol, diacetone alcohol, 2- butoxyethanol and cyclohexanole; glycols that are in a liquid state at room temperature, such as ethylene glycol, propylene glycol, pentylene glycol and glycerol; propylene glycol ethers that are in a liquid state at room temperature, such as propylene glycol monomethyl ether, propylene glycol monomethyl ether acetate and dipropylene glycol mono-n-butyl ether; short chain esters with 3 to 8 carbon atoms, such as ethyl acetate, methyl acetate, propyl acetate, n-butyl acetate and isopentyl acetate; alkanes that are in a liquid state at room temperature, such as decane, heptane, dodecane and cyclohexane; cyclic aromatic compounds that are in a liquid state at room temperature such as toluene and xylene. In particular, ethanol or short chain ester is preferable due to their high level of safety to human bodies.

The cosmetic of the present invention may contain a moisturizing agent, solid oil, liquid oil, a powder, etc. as needed

Examples of the moisturizing agent include polyethylene glycol, propylene glycol, glycerin, 1,3-butylene glycol, hexylene glycol, xylitol, sorbitol, maltitol, chondroitin sulfate, mucoitinsulfuric acid, caronic acid, atelocollagen, cholesteryl-12-hydroxy stearate, sodium lactate, bile salt,dl-pyrrolidone carboxylate, a diglycerol (EO) PO adduct, a chestnut rose extract, a yarrow extract, a melilot extract, etc.

Examples of the solid oil include: hydrocarbons such as polyethylene wax, an ethylene propylene copolymer, solid paraffin wax, ceresin wax, microcrystalline wax, Fischer-Tropsch wax and montan wax; waxes such as carnauba wax, candelilla wax, beeswax, Japan wax and spermaceti; fats and oils such as cocoa butter, palm oil and beef tallow; higher fatty acids such as stearic acid, lauric acid, myristic acid and behenic acid; higher alcohols such as cetyl alchohol, stearyl alcohol, lauryl alcohol and behenyl alcohol; hydrogenated oils such as hydrogenated coconut oil and hydrogenated castor oil; esters such as methyl stearate, cetyl palmitate, pentaerythritol rosinate and propylene glycol distearate; and silicone waxes such as stearyl-modified polysiloxane and behenyl-modified polysiloxane. These solid oils may be used in a combination of two or more kinds.

Irrespective of the origin such as animal oil, vegetable oil or synthetic oil, examples of the liquid oil include hydrocarbons, fats and oils, hydrogenated oils, ester oils, aliphatic acids, higher alcohols, silicone oils, fluorine-based oils, lanolin derivatives, oil-based gelling agents, lipophilic surfactants and oil-soluble UV absorbers. Specifically, examples of the liquid oil include: hydrocarbons such as liquid paraffin, squalane, vaseline, polyisobutene and polybutene; fats and oils such as olive oil, castor oil, jojoba oil, mink oil and Macadamia nut oil; esters such as cetyl isooctanate, isopropyl myristate, isopropyl palmitate, octyldodecyl myristate, glyceryl trioctanate, diglycerol diisostearate, diglycerol triisostearate, glyceryl tribehenate, pentaerythritol rosinate, neopentyl glycol dioctanate, cholesterol fatty acid ester and di(cholesteryl-behenyl-octyldodecyl) N-lauroyl-L-glutamate; aliphatic acids such as isostearyl acid and oleic acid; higher alcohols such as oleyl alcohol and isostearic alcohol; silicones such as a dimethylpolysiloxane oiligomer, a dimethylpolysiloxane high polymer, methylphenyl polysiloxane, decamethyl cyclo pentasiloxane, octamethyl cyclo tetrasiloxane, polyether-modified polysiloxane, cross-linked organopolysiloxane and fluorine-modified silicone; fluorine-based oils such as perfluoropolyether, perfluorodecane and perfluorooctane; lanoline derivatives such as lanoline acetate, lanoline fatty acid isopropyl arid lanoline alcohol, oil-based gelling agents such as dextrin fatty acid ester, sucrose fatty acid ester, starch fatty acid ester, aluminum 12-hydroxystearate and calcium stearate; and oil-soluble UV absorbers such as ethyl p-aminobenzoate, p-methoxyormamic-2-ethylhexyl, 4-tert-butyl-4'-methoxy-dibenzoylmethane and oxybenzone. These liquid oils may be used in a combination of two or more kinds.

The shape of the powder is not particularly limited and it may have a spherical shape, a plate shape or a needle shape. The average particle size of the powder may be smaller than or the same as that of the black bright pigment of the present invention. Further, the powder may have a porous structure or a nonporous structure. In terms of favorable spreadability and feeling in use as the cosmetic, the average particle size of the powder is preferably 0.05 to 50 µm and more preferably 0.05 to 10 µm.

Examples of the powder include inorganic powders, bright powders, organic powders, pigmentary powders and composite powders. These powders may be used in a combination of two or more kinds and the surface of the powder may be treated with metallic oxide, metallic hydroxide, a fluorine compound, silicone oil, metallic soap, wax, fats and oils, hydrocarbon, etc.

Examples of the inorganic powders include a titanium oxide powder, a black titanium oxide powder, an iron blue powder, an ultramarine blue powder, a red oxide powder, a yellow iron oxide powder, a black iron oxide powder, a zinc oxide powder, an aluminum oxide powder, a magnesium oxide powder, a zirconium oxide powder, a magnesium carbonate powder, a calcium carbonate powder, a chrome oxide powder, a chromium hydroxide powder, a carbon black powder, an aluminum silicate powder, a magnesium silicate powder, an aluminum-magnesium silicate powder, a mica powder, a synthetic mica powder, a synthetic sericite powder, a sericite powder, a talc powder, a kaoline powder, a silicic anhydride powder, a silica bead powder, a silicon carbide powder, a barium sulfide powder, a bentonite powder, a smectite powder and a boron nitride powder.

Examples of the bright powders include a bismuth oxychloride powder, a natural mica-titanium powder (natural mica coated with titanium dioxide), a natural mica powder coated with iron oxide, a natural mica-titanium powder coated with iron oxide, a natural mica-titanium powder treated with an organic pigment and an aluminum powder.

Examples of the organic powders include a nylon powder, polymethyl methacrylate, an acrylonitrile-methacryl copolymer powder, a vinylidene chloride-methacryl copolymer powder, a polyethylene powder, a polymethyl celsesquioxane powder, an organopolysiloxane elastomer powder, a urethane powder, a wool powder, a silk powder, crystalline cellulose, and N-acyllysine.

Examples of the pigmentary powders include an organic tar-based pigment and a lake pigment of organic dye.

Examples of the composite powders include a reduced titanium natural mica powder coated with fine titanium oxide particles, a titanium natural mica powder coated with fine zinc oxide particles, a titanium natural mica powder coated with barium sulfate, a titanium oxide-containing silicon dioxide powder and a zinc oxide-containing silicon dioxide powder.

The cosmetic of the present invention may contain a surfactant, an antioxidant, perfume, an antiseptic, water, glycerin, polyhydric alcohol such as 1,3-butylene glycol, lower alcohol or a cosmetic component as needed.

Although the preferred content of the black bright pigment of the present invention in the cosmetic has been described above, the content of each component in the cosmetic is not limited in the present invention and it may be similar to that in a variety of conventionally known cosmetics.

### Artificial Marble Molded Product

It is preferable that the artificial marble molded product has a three-layer structure composed of a transparent gelcoat layer disposed at the top, an intermediate layer containing the black bright pigment of the present invention and disposed below the transparent gelcoat layer, and an artificial marble layer containing a colored aggregate and disposed below the intermediate layer. Since the top layer of such artificial marble is a transparent gelcoat layer, the appearances of the layers lower than the gelcoat layer are reflected as the appearance of the artificial marble. Thus, the artificial marble molded product has glittering and presents a marble-like appearance with high blackness due to the glittering brought by the black bright pigment contained in the intermediate layer and the color tone of the artificial marble layer.

The transparent gelcoat layer preferably has a thickness of 0.3 to 0.7 mm, taking into consideration the sense of depth and a reduction in visible light transmittance. Although the composition of the transparent gelcoat layer is not particularly limited, a thermosetting resin is preferable because it is easy to handle and has high process moldability Specifically, examples of such a thermosetting resin include an unsaturated polyester resin, a vinyl ester resin, an epoxy resin, a urethane resin, a phenol resin, a mixture and a modified product (e.g., a modified product obtained by changing the end group of an unsaturated polyester resin to acrylic) thereof An unsaturated polyester resin is particularly preferable because of its high transparency, cheapness and availability.

The intermediate layer is for providing the marble-like appearance with brightness and it should not cover the pattern of the artificial marble layer. Therefore, the intermediate layer needs to have at least visible light transmittance. As long as the appearance is not compromised, a layer other than the intermediate layer may be disposed between the transparent gelcoat layer and the artificial marble layer. For example, in addition to the intermediate layer, a colored film having high visible light transmittance may be disposed between the transparent gelcoat layer and the artificial marble layer. In this case, the color tone of the artificial marble molded product can be easily adjusted.

The intermediate layer preferably has a thickness of 0.05 to 1 mm and includes a thermosetting resin with high visible light transmittance as its component. Further, the intermediate layer may contain a curing agent and an accelerating agent, in addition to the black bright pigment. The intermediate layer may contain a thickener, a thixotropic agent, an antifoaming agent or a property improver as needed. Furthermore, the intermediate layer may contain one or more pigments selected from the group consisting of a coloring pigment, other metallic pigment (aluminum pigment and iron-oxide pigment) and an interference color pigment (mica coated with metal oxide, etc.) in an amount that does not compromise the color tone of the base (the artificial marble layer).

The artificial marble layer preferably has a thickness of 3 to 25 mm. It may contain a thermosetting resin as its main component and an aggregate, a promoter, a curing agent and a colorant as other components. For the thermosetting resin, a resin similar to that used for the transparent gelcoat layer may be used. For example, an unsaturated polyester resin is preferable. Examples of the aggregate include inorganic materials such as glass frit, white marble, aluminium hydroxide, calcium oxide and a silica powder and organic materials such as a thermoplastic polyester resin. The artificial marble layer further may contain glass fibers as a reinforcement as needed

The content of each component in each layer is not limited in the present invention, and it may be similar to that in conventionally known artificial marble molded products. However, in terms of achieving appropriate properties, high brightness and sufficient sense of depth, the content of the black bright pigment in the intermediate layer is preferably 0.01 to 10 parts by mass with respect to 100 parts by mass of the thermosetting resin.

When high strength is required for the artificial marble molded product, a fiber reinforced plastic (herein after referred to as FRP) layer may be provided on the backside (the surface opposite to the surface facing the intermediate layer) of the artificial marble layer. The FRP layer is obtained by impregnating chopped strand glass mat with a thermosetting resin, such as an unsaturated polyester resin, a vinyl ester resin or an epoxy resin, and heat-curing them. Further, by adding a colorant to the FRP layer, it is possible to provide the artificial marble molded product with a hue or it is possible to block a light beam from the backside.

The method of producing the artificial marble molded product is not particularly limited and a conventional method can be used in its entirety. For example, when producing a product with a complex shape such as a bathtub, a method such as casting or press molding can be used. Further, when producing a bathtub provided with the FRP layer, the following methods can be used. In one method (spray up method), after forming the artificial marble layer, a thermosetting resin containing chopped strand glass is sprayed on the surface of the artificial marble layer with a spray gun. In another method, after applying chopped strand glass mat impregnated with a thermosetting resin on the surface of the artificial marble layer using the hand lay up method, they are cured for an hour at 50°C. Or, prior to forming the artificial marble layer, the FRP layer can be formed in advance on the surface of the mold release material of the bathtub using the spray up method or the hand lay up method.

The inorganic base constituting the black bright pigment contained in the artificial marble molded product preferably has an average particle size of 0.01 to 2 mm and an average thickness of 0.5 to 30 µm. When the inorganic base is in such a form, the black bright pigment is less likely to be crushed during the forming process of the artificial marble molded product and presents high glittering.

### Coated Paper

The coated paper can be obtained by coating one side or the both sides of base paper with a solution obtained by adding an adhesive, the black bright pigment of the present invention and a surfactant to a solvent such as water in a single layer or two or more layers.

Examples of the adhesive include copolymer latex, starch, etc.

At least one of an antioxidant, a UV absorber, a water resistant agent, an antifungal agent, an antiseptic, an antifoaming agent, perfume and a dye may be added to the solution as needed.

The coating can be carried out by using a coater such as a blade coater, an air knife coater, a roll coater, a curtain coater, a bar coater, a gravure coater or a size press coater.

In terms of enhancing the glittering of the black bright pigment, the inorganic base constituting the black bright pigment contained in the above-described solution preferably has an average particle size of 0.01 to 2 mm and an average thickness of 0.5 to 30 µm.

The content of each component in the above-described solution is not limited in the present invention, and it may be similar to that in conventionally known solutions. However, in terms of enhancing the coating efficiency as well as improving the finished quality, the content of the black bright pigment is preferably 1 to 40 mass% and more preferably 5 to 20 mass%, for example.

Hereinafter, one example of the present invention will be described in detail with reference to Examples and Comparative Examples.

The average particle size of each of the inorganic base and the black bright pigment is measured by using a laser diffraction type particle size meter and it corresponds to 50% of the cumulative volume in the particle size distribution (D50). The largest particle size of the black bright pigment corresponds to 100% of the cumulative volume in the particle size distribution (D100). In Examples and Comparative Examples, a laser diffraction particle size distribution analyzer (product name: "MICROTRAC HRA", manufactured by NIKKISO CO., LTD) is used to measure the particle size distributions.

The average thickness of the inorganic base is determined by measuring the thickness of 100 inorganic base grains and determining the average. The thickness of each inorganic base is determined by measuring an optical-path difference between a direct light beam (a light beam not influenced by a phase object) and a light beam that has transmitted the inorganic base, with use of an interference microscope.

The average width of the crystal dendrites is measured as follows. The surface of the black bright pigment is observed under a magnification of 200,000x using FE-SEM (Field Emission Scanning Electron Microscope: S-4700, manufactured by Hitachi High-Technologies) to measure the largest width of 10 crystal dendrites, and the average of the obtained measured values is determined.

The average particle size of the nanoparticles is measured as follows. The surface of the black bright pigment is observed under a magnification of 200,000x using FE-SEM (Field Emission Scanning Electron Microscope: S-4700, manufactured by Hitachi High-Technologies) to measure the particle size of 30 nanoparticles, and then the average of the obtained measured values is determined.

### Example 1

Similarly to the example shown in FIG. 1, a black bright pigment of Example 1 had a structure in which an inorganic base was covered with a coating. The black bright pigment of Example 1 was produced as follows.

### Inorganic Base

As the inorganic base, a scaly glass base having an average particle size (D50) of 38 µm and an average thickness of 1.3 µm was prepared in the following manner. First, C glass was melted at 1200°C, and the molten glass was streched, while being expanded in a hollow shape, to be thin and then was cooled to be solidified. The obtained glass film was crushed by a roller to obtain flake glass and the flake glass was crushed using a jet mill crusher to obtain the scaly glass base.

### Pretreatment of Scaly Glass Base

1.2 kg of the scaly glass base (C glass composition, average thickness: 1.3 µm, average particle size: 38 µm) was added to 8.4 L of ion-exchange water, and they were stirred by a stirrer. During the stirring, dilute hydrochloric acid was added to the solution so as to adjust its pH to 3.0. After adding 1.7 L of an aqueous solution of 0.5 mass% of tin(II) chloride at room temperature to the obtained slurry solution, the obtained mixture was adjusted to have pH of 2.0 with dilute hydrochloric acid. Then, the mixture slurry solution was stirred for 5 minutes. Thereafter, the mixture slurry solution was filtered under reduced pressure to recover the scaly glass base and the scaly glass base was washed with ion-exchange water.

9.6 L of a plating solution described in [Silver Electroless Plating Solution Composition A] below was heated to 30°C while being stirred 1.2 kg of the scaly glass that had been subjected to the pretreatment as described above was dispersed in the plating solution to obtain a suspension. The suspension was stirred for 30 minutes. The suspension was filtered under reduced pressured to collect solids (product), and then the solids were washed with pure water. After being dried at a constant temperature of 180°C, the solids further were fired in an electric heating furnace at 400°C for two hours. The obtained black bright pigment was sieved using a stainless mesh having an opening of 75 µm to obtain a black bright pigment with a largest particle size of 200 µm or less. The black bright pigment presented a black color with no red tinge.

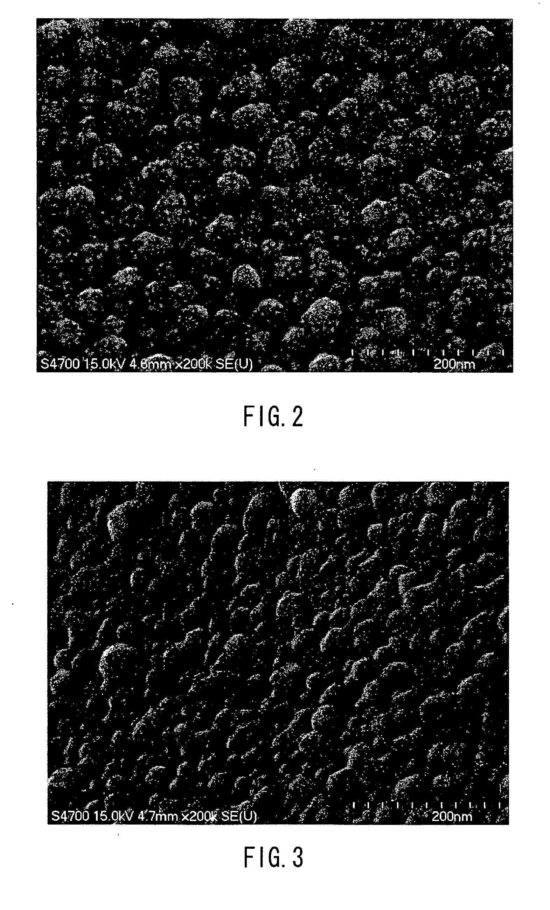

FIG. 2 is an image of the surface of the black bright pigment of Example 1, taken with a scanning electron microscope. As shown in FIG. 2, the surface of the scaly glass base was covered with the coating, and the coating contained nanoparticles with asperities, like a small candy made by crystallizing sugar, formed by the growth of crystal dendrites. The average width of the crystal dendrites was 5 nm.

The average particle size of the nanoparticles contained in the coating covering the scaly glass base was 30 nm.

### Silver Electroless Plating Solution CompositionA

silver nitrate 14 g/L
sodium hydroxide 2.5 g/L
25% ammonium hydroxide solution 26 g/L
grape sugar 7.5 g/L
ethylene diamine 18 g/L
water

### Example 2

In Example 2, the surface of a scaly glass base was covered with a coating composed of a silver-gold-pallackum alloy. The composition of the silver-gold-palladium alloy was 99.2% of silver, 0.6 atomic% of gold and 0.2 atomic% of palladium.

In Example 2, similarly to Example 1, the scaly glass base (C glass composition, average thickness: 1.3 µm, average particle size: 38 µm) was used as the scaly inorganic base. The scaly glass base was subjected to the pretreatment, and then 9.6 L of a plating solution described in [Silver Electroless Plating Solution Composition A] above was heated to 30°C while being stirred. 1.2 kg of the pretreated scaly glass was dispersed in the plating solution to obtain a suspension, and the suspension was stirred for 15 minutes.

Next, 3 g of an aqueous solution of sodium gold sulfite (100 g/L as gold), 2 g of an aqueous solution of 1% diamino palladium nitrite and 75 g of an aqueous solution of 3% sodium L-ascorbate were added to the suspension and the suspension was stirred for 15 minutes. Thereafter, the suspension was filtered under reduced pressured to collect solids (product), and then the solids were washed with pure water. After being dried at a constant temperature of 180°C, the solids further were fired in an electric heating furnace at 500°C for two hours. The obtained black bright pigment was sieved using a stainless mesh having an opening of 75 µm to obtain a black bright pigment with a largest particle size of 200 µm or less. The black bright pigment presented a black color with no red tinge.

In the black bright pigment of Example 2, similarly to the black bright pigment of Example 1, the surface of the scaly glass base was covered with the coating, and the coating contained nanoparticles with asperities, like a small candy made by crystallizing sugar, formed by the growth of crystal dendrites. The average width of the crystal dendrites was 7 nm. The average particle size of the nanoparticles contained in the coating was 34 nm.

### Comparative Example 1

A bright pigment of Comparative Example 1 had a structure in which the surface of a scaly glass base was covered with a nickel-phosphorus alloy. The composition ratio P/Ni (weight ratio) of the nickel-phosphorus alloy was 0.1.

### Pretreatment of Scaly Glass Base

The scaly glass base was same as that in Example 1. 1.2 kg of the scaly glass base (C glass composition, average thickness: 1.3 µm, average particle size: 38 µm) was added to 4 L of ion-exchange water, and they were stirred by a stirrer to obtain a slurry solution. After adding 0.3 L of an aqueous solution of 0.5 mass% tin(II) chloride at room temperature to the slurry solution, the obtained mixture was stirred for 5 minutes. Thereafter, the mixture was filtered under reduced pressure to recover the scaly glass base and the scaly glass base was washed with ion-exchange water. Next, the scaly glass base was dispersed in 4 L of pure water to obtain a slurry solution. After adding 0.01 L of an aqueous solution of 2 mass% of palladium(II) chloride at room temperature to the slurry solution, the obtained mixture was stirred for 5 minutes. Thereafter, the mixture was filtered under reduced pressure to recover the scaly glass base and the scaly glass base was washed with ion-exchange water.

20 L of a plating solution described in [Electroless Plating Solution Composition] below was heated to 70°C while being stirred 1.2 kg of the scaly glass that had been subjected to the pretreatment as described above was dispersed in the plating solution to obtain a suspension. The suspension was stirred for 30 minutes. The suspension was filtered under reduced pressured to collect solids (product), and then the solids were washed with pure water. Thereafter, the solids were vacuum dried at 150°C. The obtained black bright pigment was sieved using a stainless mesh having an opening of 75 µm to obtain a black bright pigment with a largest particle size of 200 µm or less. Although the color of the black bright pigment was black, there were also pieces having a red tinge.

In the bright pigment of Comparative Example 1, although the surface of the scaly glass base was covered with the nickel-phosphorus alloy coating, crystal dendrites as included in the bright pigment of Example 1 were not formed on the nanoparticles contained in the above coating. The average particle size of the nanoparticles contained in the coating covering the scaly glass base was 60 nm.

### Electroless Plating Solution Composition

sodium hypophosphite 25 g/L
sodium acid citrate 15 g/L
ammonium acetate 10 g/L
ammonium sulfate 5 g/L
nickel sulfate 25 g/L
lead nitrate 30 mg/L
water

### Comparative Example 2

A bright pigment of Comparative Example 2 was prepared in a manner similar to the bright pigment of Example 1, except that the firing in an electric heating furnace (400°C, two hours) was not carried out. The bright pigment of Comparative Example 2 had a structure in which the surface of a scaly glass base was covered with silver.

The color tone of the bright pigment of Comparative Example 2 was yellowish dark grey while slightly presenting a metallic luster. The color tone was less than jet-blackish color.

FIG. 3 is an image of the surface of the bright pigment of Comparative Example 2, taken with a scanning electron microscope. As shown in FIG. 3, although the surface of the scaly glass base was covered with the silver coating, crystal dendrites as included in the bright pigment of Example 1 were not formed on the nanoparticles contained in the above coating. The average particle size of the nanoparticles contained in the coating covering the scaly glass base was 28 nm.

### Comparative Example 3

A bright pigment of Comparative Example 3 had a structure in which the surface of a scaly glass base was covered with silver.

Similarly to Example 1, the bright pigment of Comparative Example 3 was prepared by using the scaly glass base (C glass composition, average thickness: 1.3 µm, average particle size: 38 µm) as the scaly inorganic base. After subjecting the scaly glass base to the pretreatment, 9.6 L of a silver plating solution descried in [Silver Electroless Plating Solution Composition B] below was heated to 30°C while being stirred. 1.2 kg of the scaly glass that had been subjected to the pretreatment was dispersed in the plating solution to obtain a suspension, and the suspension was stirred for 15 minutes.

The suspension was filtered under reduced pressured to collect solids (product), and then the solids were washed with pure water. After being dried at a constant temperature of 180°C, the solids further were fired in an electric heating furnace at 400°C for two hours. The obtained bright pigment was sieved using a stainless mesh having an opening of 75 µm to obtain a bright pigment with a largest particle size of 200 µm or less. The bright pigment presented a metallic luster of yellowish silver with strong brightness.

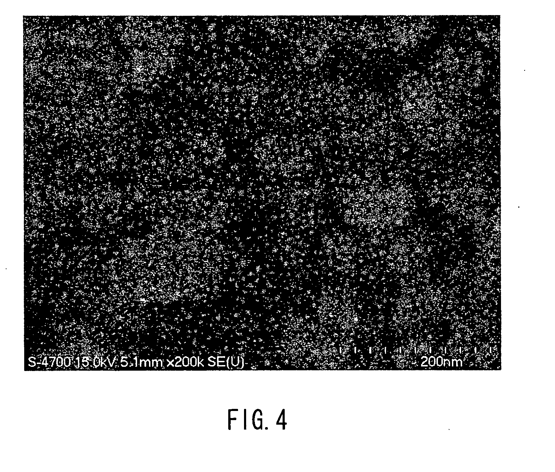

FIG. 4 is an image of the surface of the bright pigment of Comparative Example 3, taken with a scanning electron microscope. As shown in FIG. 4, unlike the coatings in the bright pigment of Examples 1 and 2, a particle shape was not clearly observed in the silver coating. However, crystal dendrites were observed on the surface of the coating. The average width of the crystal dendrites was 9 nm.

### Silver Electroless Plating Solution Composition B

silver nitrate 70 g/L
sodium hydroxide 12.5 g/L
25% ammonium hydroxide aqueous solution 130 g/L
grape sugar 37.5 g/L
ethylene diamine 90 g/L
water

### Production of Paint

Each paint was produced by mixing 78 mass% of an acrylic resin (trade name: "ACRYDIC A-322", manufactured by Dainippon Ink and Chemicals, Inc.), 16 mass% of a butyle melamine resin (trade name: "SUPER BECKAMINE L-117-60", manufactured by Dainippon Ink and Chemicals, Inc.), 6 mass% of the black bright pigment of Example 1 or 2 or Comparative Example 1, 2 or 3 and a proper amount of thinner and stirring the mixture by a stirrer. The thinner was added in an amount that made the viscosity of each paint at 20°C become 13 Pa·s. Each mass% value of the acrylic resin, the butyle melamine resin and the bright pigment was a value when the total mass of these components was 100 mass%. Further, the viscosity was a value measured using Ford Cup No. 4, manufactured by Yasuda Seiki Seisakusho, LTD.

Each paint was applied to a coating board (D-7, manufactured by Nippon Route Service Co. Ltd, intermediate coating color: N = 6.0) using a spray gun (W-100, manufactured by Anest Iwata Corp.) to form a metallic base layer.

Next, a top clear paint composition was prepared by mixing 72 mass% of an acrylic resin (trade name: "ACRYDIC A-345", manufactured by Dainippon Ink and Chemicals, Inc.), 28 mass% of a butyle melamine resin (trade name: "SUPER BECKAMINE L-117-60", manufactured by Dainippon Ink and Chemicals, Inc.) and thinner and stirring the mixture with a stirrer. The thinner was added in an amount that made the viscosity of the top clear paint composition at 20°C become 24 Pa·s. Further, the viscosity of the top clear paint composition was a value measured using Ford Cup No. 4, manufactured by Yasuda Seiki Seisakusho, LTD.

The top clear paint composition was applied to each metallic base layer using a spray gun (W-100, manufactured by Anest Iwata Corp.) and was fired (140°C, 30 minutes) to form a top clear layer. Samples A for evaluation were obtained. Regarding the thickness of each coating after the firing, the metallic base layer had a film thickness of 15 µm and the top clear layer had a film thickness of 30 µm.

### Evaluation of Samples A

The luminance (L*) and the color saturation (a* and b*) of each sample A were measured using a colorimeter (CR-400, manufactured by Konica Minolta Sensing Inc.). The results are shown in Table 1.

**[TABLE 1]**

| | Ex.1 | Ex. 2 | Comp. Ex. 1 | Comp.Ex.2 | Comp. Ex. 3 |
|---|---|---|---|---|---|
| L* | 40.10 | 39.5 | 42.6 | 48.1 | 77.4 |
| a* | -0.34 | -0.09 | 0.67 | -0.16 | -1.29 |
| b* | -0.85 | -0.18 | 0.75 | 2.68 | 11.3 |

When L* is closer to 0 and both a* and b* are negative, it means that the blackness is high. As can be seen from the results shown in Table 1, in Examples 1 and 2, L* is close to 0 than in Comparative Examples 1 to 3 and a* and b* are both negative. From these results, it can be understood that the bright pigment of Example 1 is prevented from taking on a tinge such as red or yellow and has high blackness than those of Comparative Examples 1 to 3.

### Production of Gravure Ink

Each gravure ink was produced by mixing 5 parts by mass of the bright pigment of Example 1 or 2 or Comparative Example 1, 2 or 3 and 95 parts by mass of a gravure ink vehicle mixed with the following components.

### Composition of Gravure Ink Vehicle

cellulose nitrate (RS1/4) 5.5 parts by mass
ethyl acetate 4.0 parts by mass
isopropyl alcohol 2.0 parts by mass
ethanol 20.0 parts by mass
plasticizer 1.0 part by mass

Each gravure ink was applied to a transfer film and dried at 50 to 60°C. Samples B were obtained

### Evaluation of Samples B

The luminance (L*) and the color saturation (a* and b*) of each sample B were measured using a colorimeter (CR-400, manufactured by Konica Minolta Sensing Inc). The results are shown in Table 2.

**[TABLE2]**

| | Ex.1 | Ex. 2 | Comp. Ex. 1 | Comp.Ex.2 | Comp. Ex. 3 |
|---|---|---|---|---|---|
| L* | 39.5 | 38.3 | 41.2 | 49.1 | 78.4 |
| a* | -0.48 | -0.10 | 0.77 | -0.25 | -0.82 |
| b* | -0.83 | -0.43 | 0.74 | 3.26 | 11.9 |

As can be seen from the results shown in Table 2, in Examples 1 and 2, L* is close to 0 than in Comparative Examples 1 to 3 and a* and b* are both negative. From these results, it can be understood that the bright pigments of Examples 1 and 2 are prevented from taking on a tinge such as red or yellow and have high blackness than those of Comparative Examples 1 to 3.

### Production of Cosmetic Samples

By using the black bright pigment of Example 1 or Comparative Example 1, aqueous eyeliners having the following composition were each prepared as follows.

The components described in (Composition of Aqueous Eyeliner) below except the acrylic alkyl copolymer emulsion were mixed and dispersed with each other at 80°C to obtain a mixture. Next, the acrylic alkyl copolymer emulsion was gradually added to the mixture. After being stirring by a mixer, the mixture was cooled to room temperature to obtain the aqueous eyeliner.

### Composition of Aqueous Eyeliner

acrylic alkyl copolymer emulsion (solids: 40%) 20.0 parts by mass
hydroxypropyl cellulose 2.0 parts by mass
carboxyvinyl polymer 0.5 parts by mass
1,3-butylene glycol 5.0 parts by mass
propylene glycol 2.0 parts by mass
antiseptic 0.2 parts by mass
purified water proper amount bright pigment of any one of Examples 1 and 2 and Comparative Examples 1 to 3 10 parts by mass

### Evaluation Method of Cosmetic Samples

20 female panelists were asked to actually use the cosmetic samples and compare the cosmetics respectively containing the black bright pigment of Examples 1 and 2 and the black bright pigment of Comparative Examples 1 to 3 from each other in terms of the cosmetic effect of enhancing the outlines of eyes. The results of the sensory test are shown in Table 3.

**[TABLE 3]**

| | Ex.1 | Ex. 2 | Comp. Ex. 1 | Comp. Ex.2 | Comp. Ex.3 |
|---|---|---|---|---|---|
| Very effective | 7 | 9 | 1 | 0 | 0 |
| Effective | 12 | 11 | 11 | 5 | 1 |
| Not effective | 1 | 0 | 8 | 15 | 19 |

As described above, according to the present invention, a black bright pigment having high blackness and a cosmetic, paint, ink, a resin composition, etc. including the black bright pigment can be provided.

### Industrial Applicability

Since the black bright pigment of the present invention presents high blackness, it is particularly useful as materials for a black cosmetic such as eyeliner, black paint, black ink or a resin composition used for forming black resin molded products.

## Claims

1. A black bright pigment comprising:
a scaly inorganic base; and
a coating covering the inorganic base and containing nanoparticles including a pure substance of noble-metal other than copper or a noble-metal alloy (free of copper),
wherein, the nanoparticles include crystal dendrites.

2. The black bright pigment according to claim 1, wherein the nanoparticles have an average particle size of 20 to 50 nm.

3. The black bright pigment according to claim 1, wherein the crystal dendrites have an average width of 10 nm or less.

4. The black bright pigment according to claim 1, wherein the pure substance of noble metal or noble metal contained in the noble-metal alloy is at least one selected from the group consisting of silver, gold, platinum, palladium and iridium.

5. The black bright pigment according to claim 1, wherein the inorganic base includes at least one selected from the group consisting of glass, natural mica, synthetic mica, silica and alumina.

6. A cosmetic comprising the black bright pigment according to any one of claims 1 to 5.

7. Paint comprising the black bright pigment according to any one of claims 1 to 5.

8. Ink comprising the black bright pigment according to any one of claims 1 to 5.

9. A resin composition comprising the black bright pigment according to any one of claims 1 to 5.

10. A method of producing a black bright pigment, comprising the steps of
coating a scaly inorganic base with a metal-containing film including a pure substance of noble metal other than copper or a noble-metal alloy (free of copper); and
heating the inorganic base coated with the metal-containing film in an atmosphere of 350 to 550°C to make the metal-containing film into a coating containing nanoparticles having crystal dendrites on surfaces thereof

11. The method of producing a black bright pigment according to claim 10, wherein the nanoparticles have an average particle size of 20 to 50 nm.

12. The method of producing a black bright pigment according to claim 10, wherein the crystal dendrites have an average width of 10 nm or less.

13. The method of producing a black bright pigment according to claim 10, wherein the pure substance of noble metal or noble metal contained in the noble-metal alloy is at least one selected from the group consisting of silver, gold, platinum, palladium and iridium.

14. The method of producing a black bright pigment according to claim 10, wherein the inorganic base includes at least one selected from the group consisting of glass, natural mica, synthetic mica, silica and alumina.
